# EUROPÄISCHE PATENTANMELDUNG

(11) **EP 2 158 936 A2**
(43) Veröffentlichungstag der Anmeldung: **03.03.2010**
(21) Anmeldenummer: 09008644.8
(22) Anmeldetag: 02.07.2009
(51) Int. Cl.: A61N 1/05

(54) **Medizinischer Katheter mit mehreren Polen oder Elektroden**

(30) Priorität: 05.07.2008 DE 102008032500
(71) Anmelder: Osypka, Peter, 79618 Rheinfelden-Herten (DE)
(72) Erfinder: Osypka, Peter, 79618 Rheinfelden-Herten (DE)
(74) Vertreter: Maucher, Wolfgang

(57) **Zusammenfassung**

Ein medizinischer Katheter (1) weist an seinem distalen Ende (2) mehrere axial nebeneinander angeordnete Pole (3) oder Elektroden auf, die zum Stimulieren und/oder aufnehmen von Potentialen aus einem Organ des menschlichen Körpers dienen. Dabei ist eine Wendel aus so vielen parallel verlaufenden elektrisch leitenden Windungen vorgesehen, wie Pole (3) vorhanden sind. Die Windungen sind blank, so dass der Gesamtquerschnitt der Wendel und damit des Katheters (1) kleingehalten werden kann, und zwischen den Windungen, die die Pole (3) bilden, verläuft eine Isolierung (4) beispielsweise in Form einer Kunststoffschnur. Somit ist die Außenabmessung des Katheters (1) relativ gering und darüber hinaus hat sein distales Ende (2) eine gute Flexibilität (Fig. 1).

## Beschreibung

Die Erfindung betrifft einen medizinischen Katheter mit mehreren axial nebeneinander oder hintereinander angeordneten Polen oder Elektroden zum Stimulieren und/oder zum Aufnehmen von Potentialen aus einem Organ des menschlichen Körpers, zum Beispiel aus dem Herzen, aus dem Gehirn oder aus dem Gehör, mit einer aus mehreren parallel verlaufenden elektrisch leitenden Windungen gebildeten Wendel, wobei jeweils eine Windung zu einem der am distalen Ende des Katheters angeordneten Pole führt.

Aus DE 44 25 195 C1 ist ein derartiger medizinischer Katheter mit mehreren Polen oder Elektroden bekannt. Die axial hintereinander angeordneten Elektroden sind dabei an einer isolierten Metallwendel angeordnet, die distal an bestimmten Stellen abisoliert ist. Dies ergibt jedoch nur Pole mit relativ geringer Oberfläche, so dass unter Umständen aufgrund anatomischer Abweichungen sogar die Kontaktgabe gefährdet sein kann.

Bei einer aus der Praxis bekannten bipolaren Elektrode ist eine Wendel geringeren Querschnitts für den einen Pol und eine Wendel größeren Querschnitts für den anderen Pol vorgesehen, so dass eine koaxiale und konzentrische Anordnung dieser Wendeln erforderlich ist, um lediglich zwei Pole zu erhalten.

Es besteht deshalb die Aufgabe, einen Katheter der eingangs genannten Art zu schaffen, bei welchem mehrere Pole auf einem im wesentlichen übereinstimmenden Umfang angeordnet sein können, also Wendeln unterschiedlicher Durchmesser vermieden werden, wobei dennoch jeder Pol eine vergleichsweise große Oberfläche haben können soll.

Zur Lösung dieser Aufgabe ist der eingangs definierte Katheter **dadurch gekennzeichnet, dass** die Windungen zur Bildung der Pole am distalen Ende des Katheters nicht abzuisolierende blanke Windungen sind und dass zwischen den blanken Windungen zumindest im Bereich der Pole eine Isolierung angeordnet ist, die ihrerseits durch zwischen den blanken Windungen verlaufende Windungen aus isolierendem Werkstoff oder durch gewundene Kunststoffschnüre gebildet ist.

Auf diese Weise stehen Pole zur Verfügung, die über den gesamten Umfang des Katheters reichen können, wobei es sogar möglich ist, dass ein derartiger Pol auch über mehrere Windungen reicht, also eine große Ausdehnung haben kann. Die zwischen den blanken Windungen verlaufenden Windungen aus isolierendem Werkstoff oder die dort angeordneten gewundenen Kunststoffschnüre ergeben eine besonders einfache Art der Isolierung, durch welche sichergestellt sein kann, dass die nebeneinander parallel verlaufenden blanken Windungen für die verschiedenen Pole nicht in elektrischen Kontakt miteinander gelangen können.

Vorteilhaft ist ferner, dass eine große Anzahl von verschiedenen Polen vorgesehen ist, wobei dennoch die Herstellung einfach ist, weil die zu den Polen führenden Leitungen und Drähte als blanke oder zumindest im Polbereich blanke Windungen ausgeführt sind, zwischen denen gewundene Isolierungen verlaufen und bei denen ein Abisolieren von Drähten vermieden wird.

Für eine gezielte Anordnung der einzelnen Pole kann es zweckmäßig sein, wenn zur Bildung der Pole durch die Windungen jeweils wenigstens der am distalen Ende befindliche letzte Windungsgang zumindest teilweise oder am gesamten Umfang der Wendel blank gelassen ist oder wenn die Windungen zur Bildung der Pole über einen Bruchteil der Länge oder über die gesamte Länge der Wendel blank gelassen sind. Dadurch werden die auf den mehreren Windungen nebeneinander angeordneten Pole am distalen Ende des Katheters angeordnet und können dort mit ihrer relativ großen Oberfläche wirkungsvoll eingesetzt werden.

Die Windungen über die gesamte Länge der Wendel blank zu lassen ist durch die erfindungsgemäße Maßnahme möglich, dass zwischen den zu unterschiedlichen Polen führenden Windungen eine Isolierung angeordnet ist, so dass eine Isolierung der die Wendeln bildenden Windungen der elektrischen Leiter oder Drähte selbst entbehrlich ist.

Die zwischen den elektrisch leitenden Windungen angeordneten gewundenen Isolierungen können einen runden oder einen an die Form der Zwischenräume zwischen den die Pole bildenden Windungen angepasste Querschnittsform haben. Somit kann eine weitgehend glatte Außenseite des Katheters gebildet werden, bei welcher beispielsweise runde Drähte die elektrisch leitenden Windungen bilden und die aufgrund des runden Querschnitts dieser Drähte zwischen diesen befindlichen Zwischenräume durch eine Kunststoffschnur mit einem entsprechenden negativen Querschnitt ausgefüllt werden können.

Zweckmäßig ist es, wenn Windungen aus Drähten, Litzen, elektrisch leitenden Bändern und/oder Runddrähten gebildet und zu einer mehrgängigen Wendel geformt sind. Dadurch kann der Katheter an die anatomischen Anforderungen angepasst werden.

Eine weitere Ausgestaltung kann vorsehen, dass die Windungen in Richtung zu dem proximalen Ende des Katheters hin selbst derart mit einer Isolierung beschichtet oder überzogen sind, dass sich die Pole auf einer Meridianlinie der Wendeln in Richtung zum proximalen Ende hin nicht wiederholen. Beispielsweise könnte ein äußerer Überzug über die aus mehreren parallelen Windungen gebildeten Wendel bis zu dem distalen Endbereich reichen, in welchem die einzelnen Pole wirksam sein sollen, und demgemäß diesen Bereich freilassen.

Der Katheter kann also einen äußeren isolierenden Überzug aufweisen, der bis zu dem distalen Ende mit den Polen reicht und im Bereich der blanken Windungen oder Drähte oder Leitungen zur Bildung kleinflächiger Pole Öffnungen aufweist. Dabei kann der Überzug im Bereich der die Pole bildenden blank gelassenen Windungen am Umfang jeweils nur eine Öffnung haben und diese Öffnungen können vorzugsweise koaxial nebeneinander angeordnet sein.

Eine weitere vorteilhafte Ausgestaltung der Erfindung kann vorsehen, dass die Anzahl der Pole dadurch vergrößert ist, dass im Innenlumen der die erstgenannten Pole bildenden Windungen der Wendel Leitungen oder Drähte über das distale Ende der Wendel hinaus zu einem Träger geführt sind, der zusätzliche Pole bildende Elemente aufweist, die jeweils mit einer der Leitungen verbunden sind. Somit lässt sich das Innenlumen der den Katheter im wesentlichen bildenden Wendel, welches von den einzelnen Windungen umschlossen ist, zusätzlich ausnutzen, um weitere Pole zu bilden, ohne aber den Querschnitt des Katheters vergrößern zu müssen.

Dabei ist es günstig, wenn die zusätzlichen Pole an dem Träger in axialer Richtung des Katheters nebeneinander angeordnet sind. Dabei können Sie entweder in Reihe nebeneinander oder gegebenenfalls am Umfang relativ zueinander versetzt sein.

Der Träger kann an seiner Außenseite angeordnete Kontaktstellen oder umlaufende, zumindest bereichsweise blanke Ringe tragen, die als zusätzliche Pole dienen und mit den durch das Innenlumen der Wendel geführten Leitungen verbunden sind. Die Leitungen können also praktisch von innen her in den Träger eingeführt und dann mit den außen befindlichen Kontaktstellen oder Ringen verbunden sein.

Wenigstens ein Teil oder alle durch das Innenlumen der Wendel zu zusätzlichen am distalen Ende des Katheters vorgesehenen Polen geführte Leitungen können isoliert sein, so dass gegenseitige elektrische Kontakte vermieden werden.

Eine weitere Ausgestaltung des erfindungsgemäßen Katheters kann vorsehen, dass sein die Pole aufweisendes distales Ende zumindest über einen Teil seiner axialen Erstreckung eine abnehmende oder sich verjüngende, insbesondere konische Form aufweist. Dies erleichtert es, den Katheter in Öffnungen oder Höhlungen eines Körpers einzuführen und darüber hinaus kann er vor allem in sich verengenden Gefäßen oder Höhlungen gut wirksam werden.

Das Innenlumen der den Katheter im wesentlichen bildenden Wendel kann einen derartigen lichten Querschnitt aufweisen, dass ohne oder mit darin verlaufenden zusätzlichen Leitungen Platz für ein Stilett oder einen Mandrin oder dergleichen Führungselement vorhanden ist. Dadurch kann die Handhabung des Katheters erleichtert werden.

Eine weitere Ausgestaltung des erfindungsgemäßen Katheters kann vorsehen, dass er einen äußeren isolierenden Überzug aufweist, der im Bereich der blanken Windungen oder Drähte zur Bildung kleinflächiger Pole Öffnungen aufweist. Somit kann durch einen isolierenden Überzug auch im Bereich der Pole deren Ausdehnung am Umfang des Katheters vorgegeben und gegenüber dem Gesamtumfang verkleinert werden, wobei gleichzeitig auch durch derartige Öffnungen in einem äußeren Überzug die Lage der einzelnen Pole vorgegeben werden kann.

Dabei kann der Überzug im Bereich der die Pole bildenden blanken Windungen am Umfang jeweils nur eine Öffnung haben und diese Öffnungen können vorzugsweise koaxial nebeneinander angeordnet sein. Somit liegen alle Pole in Reihe nebeneinander. Es sind aber auch Anwendungen denkbar, bei welchen die einzelnen durch den Überzug freigelassenen Pole relativ zueinander am Umfang des Katheters verteilt sind.

Eine weitere Ausgestaltung des Katheters kann vorsehen, dass an der Außenseite der die Pole bildenden blanken Windungen elektrisch leitende oder Metallringe und zwischen diesen Isolierungen angeordnet sind, wobei eine einen Pol bildende Windung jeweils Kontakt mit einem Metallring hat. Dadurch können die aufgrund der Steigung der einzelnen Windungen schräg verlaufenden Pole am distalen Ende des Katheters über die erwähnten Metallringe zu Polen umgebildet werden, die am Umfang des distalen Endes koaxial zur Mittelachse des Katheters umlaufen. Somit ergibt eine Verdrehung des Katheters dennoch immer die gleiche relative Lage des entsprechenden Bereiches der Pole relativ zu einem Körperteil.

Vor allem bei Kombination einzelner oder mehrerer der vorbeschriebenen Merkmale und Maßnahmen ergibt sich ein medizinischer Katheter, bei welchem eine große Anzahl von verschiedenen Polen vorgesehen werden kann, wobei dennoch die Herstellung einfach ist, weil die zu den Polen führenden Leitungen und Drähte als blanke oder zumindest im Polbereich blanke Windungen ausgeführt sein können, zwischen denen entsprechend gewundene Isolierungen verlaufen, so da ein Abisolieren von Drähten nicht notwendig ist.

Nachstehend sind Ausführungsbeispiele der Erfindung anhand der Zeichnung näher beschrieben. Es zeigt in zum Teil schematisierter Darstellung:
- Fig. 1: einen Längsschnitt durch das distale Ende eines erfindungsgemäßen medizinischen Katheters mit mehreren, nämlich in diesem Falle sechzehn axial nebeneinander angeordneten Polen oder Elektroden zum Stimulieren und/oder zum Aufnehmen von Potentialen aus einem Organ mit einer aus mehreren parallel zueinander verlaufenden elektrisch leitenden Windungen gebildeten mehrgängigen Wendel, wobei jeweils eine Windung zu einem der Pole führt und die Windungen zumindest am distalen Ende des Katheters blank sind und zwischen ihnen eine Isolierung angeordnet ist,
- Fig. 2: einen Teilbereich der den Katheter im wesentlichen bildenden Wendel mit zwei parallelen Windungen und einer gegenüber Fig. 1 abgewandelten Querschnittsform der dazwischenliegenden Isolierung,
- Fig. 3: eine der Fig. 2 entsprechende Darstellung, wobei die elektrisch leitenden Windungen durch Litzen gebildet sind,
- Fig. 4: eine Anordnung, bei welcher die elektrisch leitenden Windungen durch Flachdrähte gebildet sind und die Isolierung dazwischen eine entsprechend angepasste Form hat,
- Fig. 5: einen Längsschnitt einer abgewandelten Ausführungsform, bei welcher auch das distale Ende des Katheters mit den einzelnen Polen einen Überzug aufweist, in welchem mit den blanken Wendeln in Kontakt befindliche Ringe angeordnet sind,
- Fig. 6: eine ebenfalls abgewandelte Ausführungsform, bei welcher der Überzug der Wendel bis zum distalen Ende verläuft und an den zu bildenden Polen Ausschnitte oder Öffnungen aufweist, unter denen die blanken Windungen der einzelnen Pole verlaufen,
- Fig. 7: eine Seitenansicht eines erfindungsgemäßen medizinischen Katheters etwa gemäß Fig. 6 mit dem proximalen Ende, wobei aber an dem distalen Ende zusätzliche Pole angeordnet sind,
- Fig. 8: in vergrößerter Darstellung das distale Ende des Katheters gemäß Fig. 7,
- Fig. 9: einen Längsschnitt des zusätzliche Pole aufweisenden Trägers für das distale Ende des Katheters gemäß Fig. 7 und 8 mit im Innenlumen der den Katheter bildenden Wendel verlaufenden Leitungen, die zu einzelnen Pole bildenden Metallringen an der Außenseite des Trägers verlaufen, sowie
- Fig. 10: eine Seitenansicht eines aus Kunststoff bestehenden Spritzteils, welches den Träger für zusätzliche Pole am distalen Ende des Katheters gemäß Fig. 7 bildet.

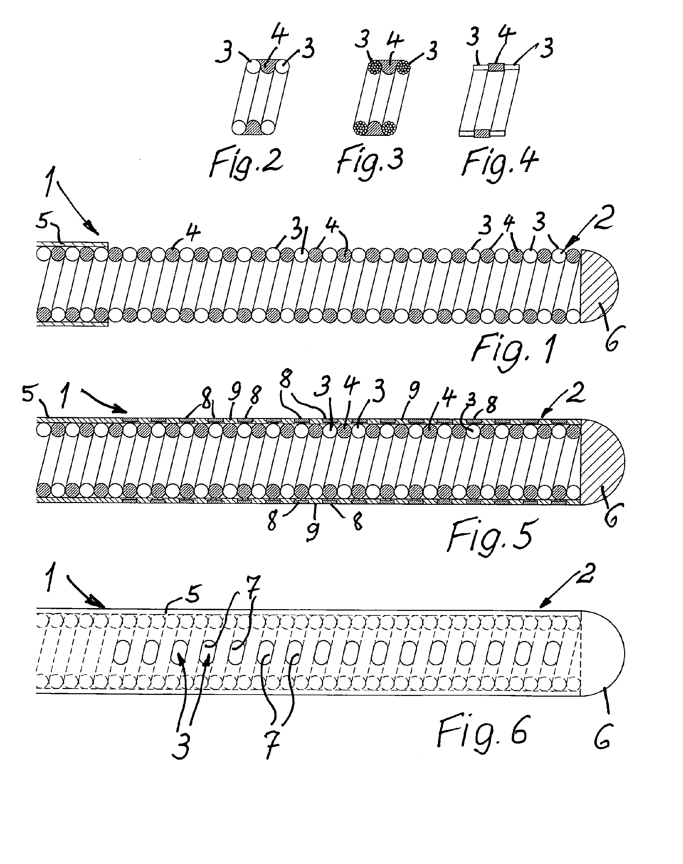

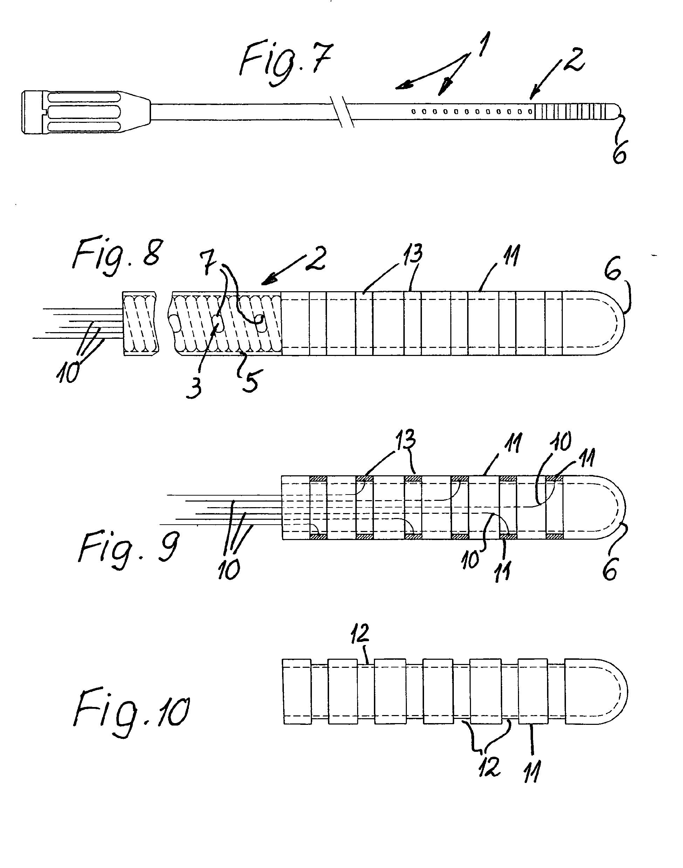

Von einem im Ganzen mit 1 bezeichneten Katheter sind in den Fig. 1, 5 und 6 jeweils Längsschnitte des distalen Endes 2 dargestellt, an welchem in diesen Ausführungsbeispielen insgesamt sechzehn Pole 3 axial hintereinander oder nebeneinander angeordnet sind, während ein abgewandeltes Ausführungsbeispiel eines Katheters 1 in Fig. 7 in einer Gesamtansicht zu sehen ist.

Allen Ausführungsbeispielen ist gemeinsam, dass eine aus mehreren parallel verlaufenden elektrisch leitenden Windungen gebildete Wendel vorgesehen ist, wobei jeweils eine Windung zu einem der am distalen Ende 2 des Katheters 1 angeordneten Pole 3 führt, wie es besonders gut in Fig. 1 , aber auch in den Fig. 5 und 6 sowie in Fig. 8 angedeutet ist.

Dabei ist in allen Ausführungsbeispielen vorgesehen, dass die Windungen zur Bildung der Pole 3 blank sind und dass gemäß den Fig. 1 bis 5 zwischen den Windungen und somit zwischen den Polen 3 eine Isolierung 4 angeordnet ist. Die Herstellung der Wendel durch blanke Windungen ist einfacher und erlaubt geringere Abmessungen, als wenn die Windungen selbst isoliert wären. Somit ergibt sich eine flexible und einfach herstellbare Wendel, die als medizinischer Katheter 1 auch eine große Anzahl von Polen 3 nebeneinander haben kann, weil die blanken Leitungen durch die zwischen ihnen verlaufenden Isolierungen 4 genauso gut oder noch besser voneinander getrennt sind als durch eine Isolierung der Windungen selbst.

An sich genügt es dabei für eine Anordnung gemäß Fig. 1 oder 5 oder 6, wenn der am distalen Ende befindliche letzte Windungsgang blank ist, denn in diesen Ausführungsbeispielen sind nur diese letzten Windungsgänge der Wendel als sechzehn Pole 3 vorgesehen.

Die Isolierung 4 zwischen den elektrisch leitenden Windungen kann ihrerseits durch Windungen aus isolierendem Werkstoff oder durch gewundene Kunststoffschnüre gebildet sein, die einen runden (Fig. 1 und 5) oder einen an die Form der Zwischenräume zwischen den die Pole 3 bildenden Windungen angepasste Querschnittsform (Fig. 2, 3 und 4) haben. Somit ergeben sich gut voneinander isolierte Pole 3 und dennoch ein distales Ende 2 des Katheters 1, welches eine hohe Flexibilität und eine sehr geringe Abmessung haben kann.

Gemäß den Fig. 1 bis 4 können die die Pole 3 bildenden Windungen aus Drähten, aus Litzen (Fig. 3), aus Bändern (Fig. 4) oder aus Runddrähten (Fig. 1, 2 und 5) gebildet und zu der erwähnten mehrgängigen Wendel geformt sein.

Dabei erkennt man besonders gut in Fig. 1, aber auch in weiteren Figuren, dass die Windungen in Richtung zu dem proximalen Ende des Katheters 1 hin durch einen Überzug 5 zumindest außenseitig derart isoliert sein können, dass sich die Pole 3 auf einer Meridianlinie der Wendel in Richtung zum proximalen Ende hin nicht wiederholen. Zwar sind die Pole 3 durch die blanken Windungen auch unter dem Überzug vorhanden, können durch diesen aber nicht wirksam sein.

Im Ausführungsbeispiel nach Fig. 6 ist der Überzug 5 auch über das distale Ende 2 fortgesetzt, reicht also bis zu einem an diesem distalen Ende stirnseitig angeordneten Abschluss 6 und weist im Bereich der jeweils letzten Windungen Öffnungen 7 auf, so dass die unter dem Überzug 5 befindlichen blanken Windungen an begrenzten Stellen als Pole 3 wirksam werden können. Dabei hat der Überzug 5 im Bereich der die Pole 3 bildenden blanken Windungen am Umfang jeweils nur eine Öffnung 7 und man erkennt in Fig. 6, dass diese Öffnungen 7 bevorzugt axial nebeneinander angeordnet sein können. Denkbar wäre natürlich auch am Umfang versetzte Öffnungen 7 und gegebenenfalls mehrere zu einem Pol 3 gehörende derartige Öffnungen 7.

In Fig. 5 erkennt man eine Anordnung, bei welcher an der Außenseite der die Pole 3 bildenden blanken Windungen elektrisch leitende oder metallische Ringe 8 und zwischen diesen Isolierungen 9 angeordnet sind, wobei eine einen Pol 3 bildende Windung jeweils Kontakt mit einem derartigen Metallring 8 hat. Auf diese Weise lassen sich die Pole 3, die im Ausführungsbeispiel nach Fig. 1 in ihrer Umfangsrichtung gleichzeitig eine axiale Erstreckung haben, weil sie als schraubenförmiger Gewindegang geformt sind, nach außen hin zu koaxialen Polen machen, deren Umfang an der selben axialen Stelle des Katheters 1 verbleibt und so auch eine beliebige gedrehte Implantation des Katheters 1 mit gleichbleibender Lage der Pole 3 erlaubt.

In den Fig. 7 bis 10 sind Weiterbildungen eines Katheters 1 näher dargestellt. Dabei ist vorgesehen, dass die Anzahl der Pole 3 dadurch vergrößert ist, dass im Innenlumen, also in dem von den Windungen der Wendel freigelassenen inneren Kanal des Katheters 1 Leitungen oder Drähte 10 über das distale Ende der Wendel hinaus zu einem mit ihr in koaxialer Richtung verbunden Träger 11 geführt sind, der zusätzliche Pole 13 bildende, noch zu erläuternde Elemente aufweist, die jeweils mit einer der Leitungen 10 verbunden sind. Dabei erkennt man vor allem in den Fig. 7 bis 9, dass die zusätzlichen Pole 13 an dem Träger 11 in axialer Richtung des Katheters 1 nebeneinander und parallel zueinander angeordnet sind.

Gemäß Fig. 8 und 9 hat der Träger 11 an seiner Außenseite angeordnete Kontaktstellen, nämlich koaxial und konzentrisch umlaufende blanke Ringe, die als zusätzliche Pole 13 dienen und mit den durch das Innenlumen der Wendel geführten Leitungen 10 jeweils einzeln verbunden sind, wie es besonders gut in Fig. 9 dargestellt ist.

Der Träger 11 ist dabei gemäß Fig. 10 ein Spritzgießteil aus Kunststoff und hat an der Stelle der zusätzlichen Pole 13 Vertiefungen 12, welche diese ringförmigen zusätzlichen Pole 13 aufnehmen. Die durch das Innenlumen der Wendel zu den zusätzlichen Polen 13 geführten Leitungen 10 sind isoliert, was nicht zu einer Vergrößerung des Querschnitts des Katheters 1 führen kann, weil sie im Inneren der Wendel verlaufen.

Das Innenlumen der Wendel weist dabei schon aufgrund der Bauweise des Katheters 1 einen derartigen Querschnitt auf, dass darin auch dann, wenn die zusätzlichen Leitungen 10 vorgesehen sind, genügend Platz für ein Stilett oder einen Mandrin oder dergleichen Führungselement vorhanden ist.

Das die Pole 3 oder 13 aufweisende distale Ende 2 könnte über einen Teil seiner Erstreckung eine konische Form haben. In den Ausführungsbeispielen ist jedoch nur ein abgerundeter Abschluss 6 vorgesehen, der ebenfalls das Einführen des Katheters in Körperöffnungen oder in Blutgefäße erleichtert.

Der medizinische Katheter 1 weist an seinem distalen Ende 2 mehrere axial nebeneinander angeordnete Pole 3 oder Elektroden auf, die zum Stimulieren und/oder aufnehmen von Potentialen aus einem Organ des menschlichen Körpers dienen. Dabei ist eine Wendel aus so vielen parallel verlaufenden elektrisch leitenden Windungen vorgesehen, wie Pole 3 vorhanden sind. Die Windungen sind blank, so dass der Gesamtquerschnitt der Wendel und damit des Katheters 1 kleingehalten werden kann, und zwischen den Windungen, die die Pole 3 bilden, verläuft eine Isolierung 4 beispielsweise in Form einer Kunststoffschnur. Somit ist die Außenabmessung des Katheters 1 relativ gering und darüber hinaus hat sein distales Ende 2 eine gute Flexibilität.

Die Verwendung blanker Leitungen oder Drähte oder Windungen zur Bildung der Pole 3 erlaubt entweder größere Querschnitte dieser Leitungen oder ergibt eine geringe Außenabmessung und eine blanke Leitung hat eine größere Flexibilität als eine Leitung mit einer Isolierbeschichtung.

Dabei verlaufen die einzelnen Windungen der mehrgängigen Wendel jeweils parallel zueinander, haben also übereinstimmende Abmessungen und bilden so eine an der Außenseite praktisch gleichmäßige Wendel.

## Patentansprüche

1. Medizinischer Katheter (1) mit mehreren axial nebeneinander oder hintereinander angeordneten Polen (3) oder Elektroden zum Stimulieren und/oder zum Aufnehmen von Potentialen aus einem Organ des menschlichen Körpers, zum Beispiel aus dem Herzen, aus dem Gehirn oder aus dem Gehör, mit einer aus mehreren parallel verlaufenden elektrisch leitenden Windungen gebildeten Wendel, wobei jeweils eine Windung zu einem am distalen Ende (2) des Katheters (1) angeordneten Pol (3) führt, **dadurch gekennzeichnet, dass** die Windungen zur Bildung der Pole (3) am distalen Ende des Katheters nicht abzuisolierende blanke Windungen sind und dass zwischen den blanken Windungen zumindest im Bereich der Pole (3) eine Isolierung (4) angeordnet ist, die ihrerseits durch zwischen den blanken Windungen verlaufende Windungen aus isolierendem Werkstoff oder durch gewundene Kunststoffschnüre gebildet ist.

2. Katheter nach Anspruch 1, **dadurch gekennzeichnet, dass** zur Bildung der Pole (3) durch die Windungen jeweils wenigstens der am distalen Ende befindliche letzte Windungsgang zumindest teilweise oder am gesamten Umfang der Wendel blank gelassen ist oder dass die Windungen zur Bildung der Pole (3) über einen Bruchteil der Länge oder über die gesamte Länge der Wendel blank gelassen sind.

3. Katheter nach einem der Ansprüche 1 oder 2, **dadurch gekennzeichnet, dass** die zwischen den elektrisch leitenden Windungen angeordneten gewundenen Isolierungen (4) einen runden oder einen an die Form der Zwischenräume zwischen den die Pole (3) bildenden Windungen angepasste Querschnittsform haben.

4. Katheter nach einem der Ansprüche 1 bis 3, **dadurch gekennzeichnet, dass** Windungen aus Drähten, Litzen, Bändern und/oder Runddrähten gebildet und zu einer mehrgängigen Wendel geformt sind.

5. Katheter nach einem der Ansprüche 1 bis 4, **dadurch gekennzeichnet, dass** die Windungen der mehrgängigen Wendel übereinstimmende Abmessungen haben und koaxial nebeneinander verlaufen.

6. Katheter nach einem der Ansprüche 1 bis 5, **dadurch gekennzeichnet, dass** er einen äußeren isolierenden Überzug (5) aufweist, der bis zu dem distalen Ende (2) mit den Polen (3) reicht und im Bereich der blanken Windungen der Drähte oder Leitungen zur Bildung kleinflächiger Pole (3) Öffnungen (7) aufweist.

7. Katheter nach einem der Ansprüche 1 bis 6, **dadurch gekennzeichnet, dass** die Windungen in Richtung zu dem proximalen Ende des Katheters (1) hin derart mit einer Isolierung beschichtet oder überzogen sind, dass sich die Pole (3) auf einer Meridianlinie der Wendel in Richtung zum proximalen Ende hin nicht wiederholen.

8. Katheter nach einem der Ansprüche 1 bis 7, **dadurch gekennzeichnet, dass** der Überzug (5) im Bereich der die Pole (3) bildenden blanken Windungen am Umfang jeweils nur eine Öffnung (7) hat und dass diese Öffnungen (7) vorzugsweise koaxial nebeneinander angeordnet sind.

9. Katheter nach einem der Ansprüche 1 bis 8 **dadurch gekennzeichnet, dass** an der Außenseite der die Pole (3) bildenden blanken Windungen am distalen Ende (2) elektrisch leitende oder metallische Ringe (8) und zwischen diesen Isolierungen (9) angeordnet sind, wobei eine einen Pol (3) bildende Windung jeweils Kontakt mit einem insbesondere koaxial angeordneten Ring (8) hat.

10. Katheter nach einem der vorstehenden Ansprüche, **dadurch gekennzeichnet, dass** die Anzahl der Pole (3) **dadurch** vergrößert ist, dass im Innenlumen der die erstgenannten Pole (3) bildenden Windungen der Wendel Leitungen (10) oder Drähte über das distale Ende der Wendel hinaus zu einem Träger (11) geführt sind, der zusätzliche Pole (13) bildende Elemente aufweist, die jeweils mit einer der Leitungen (10) verbunden sind.

11. Katheter nach Anspruch 10, **dadurch gekennzeichnet, dass** die zusätzlichen Pole (13) an dem Träger (11) in axialer Richtung des Katheters (1) nebeneinander angeordnet sind.

12. Katheter nach Anspruch 10 oder 11, **dadurch gekennzeichnet, dass** der Träger (11) an seiner Außenseite angeordnete Kontaktstellen oder umlaufende, zumindest bereichsweise blanke Ringe trägt, die als zusätzliche Pole (13) dienen und mit den durch das Innenlumen der Wendel geführten Leitungen (10) insbesondere von dem hohlen Innenraum des Trägers (11) aus verbunden sind.

13. Katheter nach einem der Ansprüche 11 bis 12, **dadurch gekennzeichnet, dass** wenigstens ein Teil oder alle durch das Innenlumen der Wendel zusätzlichen, am distalen Ende des Katheters (1) vorgesehenen Polen (13) geführte Leitungen (10) isoliert sind.

14. Katheter nach einem der Ansprüche 1 bis 13, **dadurch gekennzeichnet, dass** sein die Pole (3, 13) aufweisendes distale Ende (2) zumindest über einen Teil seiner Erstreckung eine abnehmende oder sich verjüngende, insbesondere konische, oder abgerundete Form aufweist.

15. Katheter nach einem der vorstehenden Ansprüche, **dadurch gekennzeichnet, dass** das Innenlumen der Wendel und der sie bildenden Windungen einen derartigen Querschnitt aufweist, dass ohne oder mit darin verlaufenden zusätzlichen Leitungen (10) Platz für ein Stilett oder einen Mandrin oder dergleichen Führungselement vorhanden ist.
